(19) Europäisches Patentamt / European Patent Office / Office européen des brevets

(11) **EP 3 509 546 B1**

(12) **EUROPEAN PATENT SPECIFICATION**

(45) Date of publication and mention of the grant of the patent:
**12.10.2022 Bulletin 2022/41**

(21) Application number: **17768637.5**

(22) Date of filing: **08.09.2017**

(51) International Patent Classification (IPC):
***A61F 9/008*** *(2006.01)*

(52) Cooperative Patent Classification (CPC):
**A61F 9/00804;** A61F 2009/00844;
A61F 2009/00846; A61F 2009/00872

(86) International application number:
**PCT/US2017/050779**

(87) International publication number:
**WO 2018/049230 (15.03.2018 Gazette 2018/11)**

(54) **SYSTEMS FOR OBTAINING IRIS REGISTRATION AND PUPIL CENTRATION FOR LASER SURGERY**

SYSTEME ZUR ERHALTUNG DER IRISREGISTRIERUNG UND PUPILLENZENTRIERUNG FÜR LASERCHIRURGIE

SYSTÈMES D'OBTENTION D'ALIGNEMENT D'IRIS ET DE CENTRAGE DE PUPILLE POUR CHIRURGIE AU LASER

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **08.09.2016 US 201662385147 P**

(43) Date of publication of application:
**17.07.2019 Bulletin 2019/29**

(73) Proprietor: **AMO Development, LLC
Santa Ana, CA 92705 (US)**

(72) Inventors:
• **CHEN, Li
  Santa Ana, California 92705 (US)**
• **CHERNYAK, Dimitri
  Sunnyvale, California 94087 (US)**

(74) Representative: **Carpmaels & Ransford LLP
One Southampton Row
London WC1B 5HA (GB)**

(56) References cited:
**US-A1- 2008 309 870      US-A1- 2012 242 956
US-A1- 2015 148 788**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**EP 3 509 546 B1**

**Description**

CROSS-REFERENCES TO RELATED APPLICATIONS

The present application is generally related to the following : U.S. Pat. No. 7,044,602, US 2012-0242956 AI, U.S. Pat. No. 7,708,405, and U.S. Pat. No. 6,322,216 .

FIELD OF THE INVENTION

The subject matter of the present application is generally related to systems for diagnosing and/or treating a patient's vision, and more specifically, to registering a surgical eye image with two diagnostic eye images of the same eye so as to track position and torsional cyclorotation of the eye before and/or during laser eye surgery, and to determine a customized ablation profile of the patient's eye. To allow for more accurate alignment and tracking of eye movements during a laser surgical procedure, embodiments of this invention encompass systems that monitor changes of the eye during a diagnostic and/or treatment procedure, such as changes in the pupil size. The present invention is particularly useful for enhancing the accuracy and efficacy of laser eye surgical procedures, such as photorefractive keratectomy (PRK), phototherapeutic keratectomy (PTK), laser in situ keratomileusis (LASIK), and the like.

BACKGROUND

Laser eye surgical procedures benefit from precise alignment between the corneal tissues of the eye and a therapeutic laser beam.

Known laser eye surgical systems generally employ either an ultraviolet, or an infrared laser beam to remove a microscopic layer of stromal tissue from the cornea of the eye to change the cornea's contour or shape for different purposes, such as correcting myopia, hyperopia, astigmatism, and the like. Excimer lasers use ultraviolet laser beams while femtosecond and picosecond lasers use non-ultraviolet, near infrared, ultrashort pulsed laser beams. In an all-laser LASIK procedure, an ultra-short pulsed laser is used to cut a corneal flap to expose the corneal stroma, which is then photoablated with ultraviolet beams from an excimer laser. Photoablation of the corneal stroma removes a selected portion of the corneal tissue to reshapes the cornea and correct refractive errors, such as myopia, hyperopia, astigmatism, and the like.

Typically, the laser beam removes a selected portion of the corneal tissue to correct refractive errors of the eye. Ultraviolet laser ablation results in photodecomposition of the corneal tissue, but generally does not cause significant thermal damage to adjacent and underlying tissues of the eye. The irradiated molecules are broken into smaller volatile fragments photochemically, directly breaking the intermolecular bonds.

To determine the precise contours of the cornea that can be ablated or otherwise treated with a laser, laser eye surgical systems often rely on a diagnostic refractive map of the patient's cornea. For instance, wavefront technology, such as a Shack Hartmann sensor, measures and maps ocular aberrations of the eye, typically when the pupil is relatively large. The map is then used to create an ablation pattern, which includes the positions as well as the depths of the proposed corneal ablations for correcting the aberrations.

Precise alignment of the corneal tissues and the therapeutic laser beam is highly beneficial for the procedure. To ensure proper alignment between the corneal ablation pattern and the surface of the cornea, systems rely on a variety of systems and methods, including the use of moveable apertures, controlled scanning systems, eye movement tracking mechanisms, and the like. But, these systems are generally adapted for use while the patient is awake. To adjust for movement of the eye during a procedure, tracking systems identify and track a reference feature of the eye, which may include any one or more of a pupil, an iris feature, a boundary of the iris and the sclera, and/or the location of the pupil center. During the procedure, alignment between the eye and the therapeutic laser beam can be further enhanced by having the patient focus on a fixation target, such as a fixation light.

In some instances, during the wavefront measurement exam, the patient is in a seated position and the eye is under scotopic illumination. During surgery, however, the patient is lying down in a supine position and the eye is under photopic illumination. The changes in position may affect alignment. Specifically, the eye may undergo cyclotorsional rotation when the orientation of the patient's body changes between the wavefront exam and surgery, and the pupil center may shift with respect to the surface of the cornea as the pupil changes size under scotopic illumination and photopic illumination. Iris registration (IR) may be used to compensate for the pupil center shift and cyclorotational rotation of the eye between the wavefront exam and the surgery.

Current IR methodology uses one scotopic image (SI) of a patient's eye captured by a diagnostic device as a reference to register the photopic laser image (LI) of the patient's eye captured by a laser system, and calculates the pupil center shift and cyclotorsional angle (CT) of the patient's eye under the laser system. It detects the CT angle offset of the laser image compared to the reference image SI by identifying matching blocks from the two images. However, distorted iris features caused by pupil dilates and constricts may cause IR failure with the current method due to lack of matching blocks identified from the two images.

Eye tracking systems are known from US 2012/242956 A1 or US 2015/148788 A1.

Even though laser scanning and eye tracking technology have provided significant benefits to refractive therapies in recent years, further improvements are still desired. For example, along with tracking overall changes in locations of the patient's eyes (such as when a patient slightly looks away from a fixation target), more recently developed systems have sought to both register a treatment with the eye, and track an orientation of the eye during laser eye surgery (particularly the torsional orientation of the eye about the optical viewing axis). While both torsional registration and tracking have been performed, development and implementation of a highly robust torsional tracking system has been found to be particularly challenging. Work in connection with the present invention has identified changes in the eye during a procedure that may play a significant role in the degradation and/or loss of tracking during a procedure. Hence, improved devices, systems, and/or methods, which alleviate and/or overcome these challenges would be beneficial.

BRIEF SUMMARY OF THE INVENTION

Hence, to obviate one or more problems due to limitations and disadvantages of the related art, an object of this disclosure is to provide improved ophthalmological systems, which can accurately register the patient's eye and monitor changes in the eye characteristics to allow for tracking of positional movement and torsional rotation of the patient's eye. These systems would be particularly useful for laser systems that employ a customized ablation patterns for therapeutic procedures such as LASIK. Additionally, the systems affect monitor in pupil size to allow for more robust and accurate tracking of positional movement and torsional rotation of the patient's eyes during other diagnostic and/or treatment procedures.

The present disclosure provides systems, which can improve iris registration and iris tracking before and during diagnostic and/or treatment procedures of the eye, particularly those for laser eye surgery. Unlike systems that take only one image of an iris for alignment purposes, the systems described here utilize various wavelength and intensity of light sources to monitor the pupil size during diagnostic measurements. Thus, images of the eye may be taken, wherein a larger amount of iris is showing, thereby resulting in better alignment.

In one aspect, the system of the present disclosure may take multiple images of the iris and pupil for alignment purposes, where the iris and pupil are different sizes, as affected by light. These images may then be sent to a laser system for alignment with the therapeutic laser before and during a surgical procedure. By monitoring pupil size in taking and using alignment images under different light conditions, embodiments of the present disclosure allow for more accurate tracking of positional movement and torsional rotations (such as cyclotorsional angle CT) of the patient's eye before and/or during a diagnostic and/or treatment procedure. Comparing the CT angle offset and the pupil center can help align the system to the eye during surgery to match the eye at the time of the diagnostic procedure.

For a further understanding of the nature and advantages of the invention, reference should be made to the following description taken in conjunction with the accompanying drawings. This summary and the following detailed description are merely exemplary, illustrative, and explanatory, and are not intended to limit, but to provide further explanation of the embodiments as claimed. Additional features and advantages of the embodiments will be set forth in the descriptions that follow, and in part will be apparent from the description, or may be learned by practice of the embodiments. The objectives and other advantages of the embodiments will be realized and attained by the structure particularly pointed out in the written description, claims and the appended drawings.

BRIEF DESCRIPTION OF THE DRAWINGS

The novel features of the embodiments are set forth with particularity in the appended claims. A better understanding of the features and advantages will be facilitated by referring to the following detailed description that sets forth illustrative embodiments using principles of the embodiments, as well as to the accompanying drawings, in which like numerals refer to like parts throughout the different views. Like parts, however, do not always have like reference numerals. Further, the drawings are not drawn to scale, and emphasis has instead been placed on illustrating the principles of the embodiments. All illustrations are intended to convey concepts, where relative sizes, shapes, and other detailed attributes may be illustrated schematically rather than depicted literally or precisely.

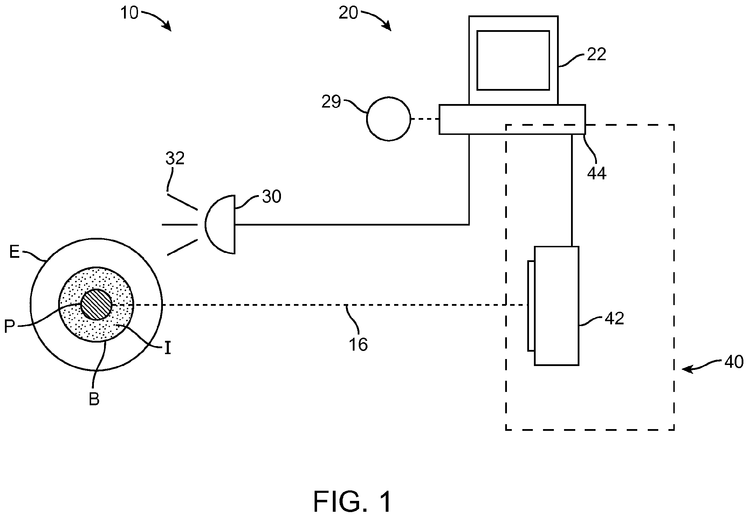

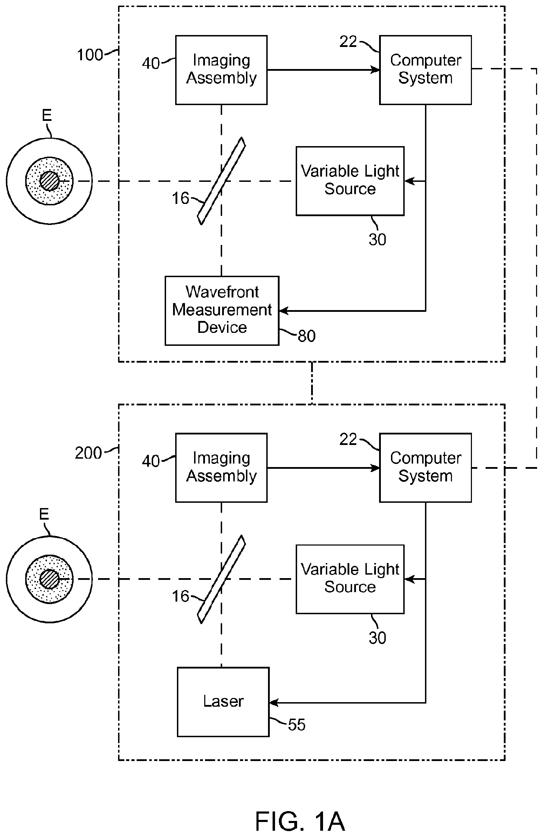

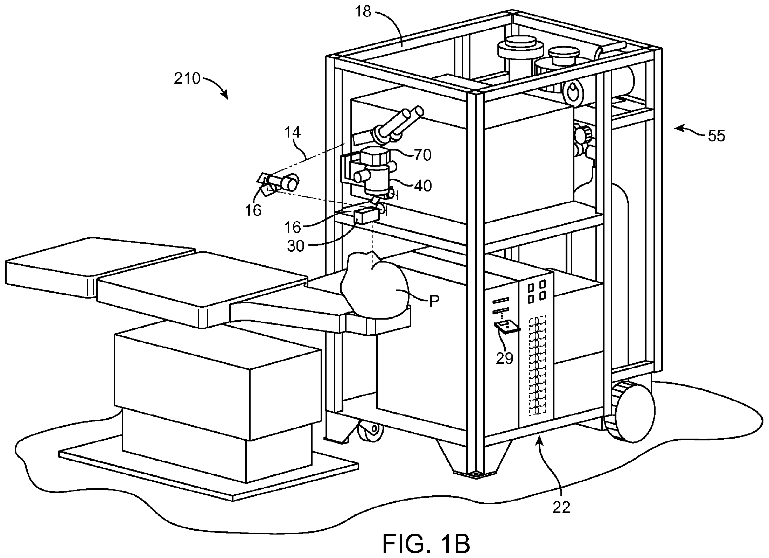

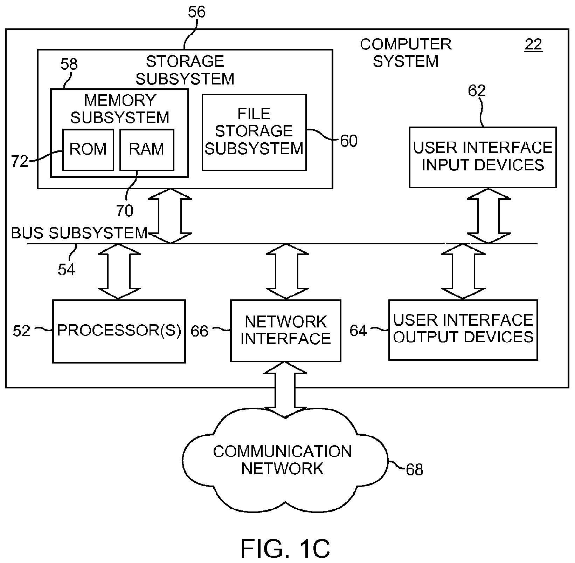

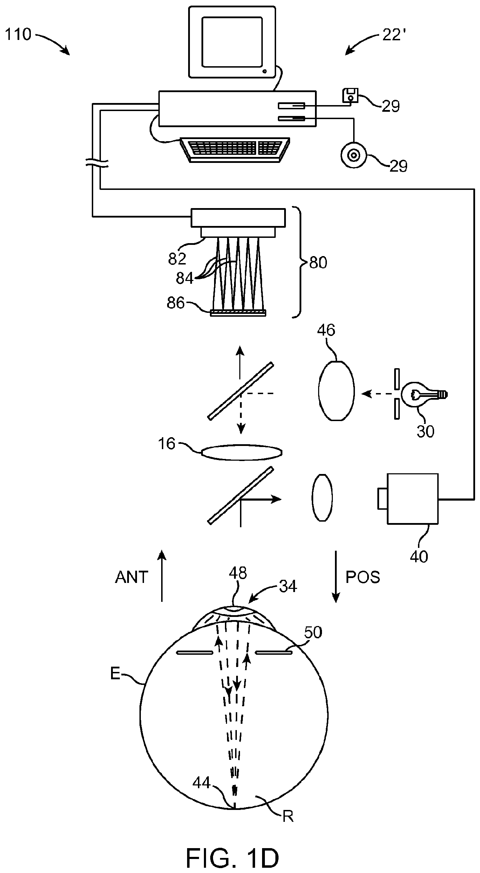

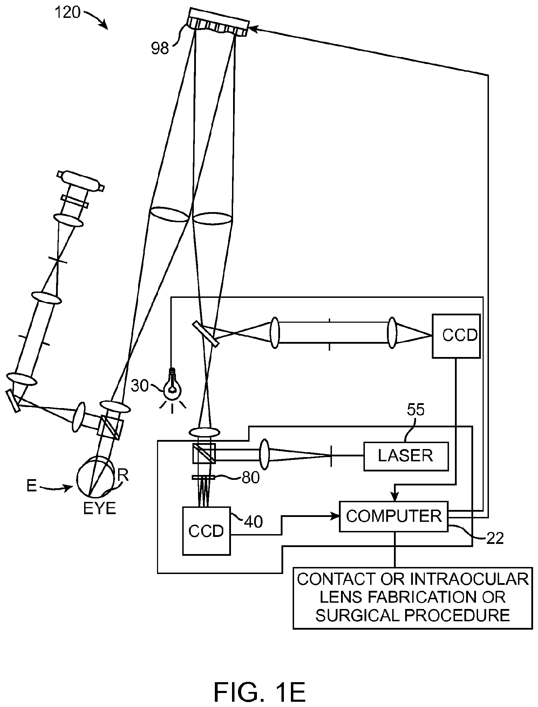

FIG. **1** schematically illustrates an embodiment of the present invention;
FIG. **1**A schematically illustrates embodiments of the present invention as a diagnostic system and as a treatment system;
FIG. **1**B illustrates a laser ablation system according to an embodiment of the present invention;
FIG. **1**C illustrates a simplified computer system according to an embodiment of the present invention;
FIG. **1**D illustrates a wavefront measurement system according to an embodiment of the present invention;

FIG. 1E illustrates another wavefront measurement system according to an embodiment of the present invention;

FIG. 2 illustrates an image captured by an image capture device of the system of FIG. 1 and also shows changes in pupil center location or pupil drift;

FIGS. 3A and 3B graphically illustrate a relationship between pupil center drift for the left eye and right eye of a patient as the pupil changes size, respectively;

FIGS. 4A and 4B schematically illustrates pupil center drift from different images by identifying centers of a pupil and an outer iris boundary;

FIG. 5 illustrates an exemplary embodiment of the present invention;

FIG. 6 illustrates change in pupil size in in scotopic and photopic eye images and laser eye images;

FIGs. 7, 8 and 9 schematically illustrate methods which can be performed with the system of the present disclosure.

DETAILED DESCRIPTION

The present embodiments generally provide improved systems for taking multiple images of an eye, wherein the eye has different pupil sizes, and using those images for alignment before and during a diagnostic and/or treatment procedure of the eye. In an exemplary embodiment, the systems provide a camera for taking an image of an eye, and an illumination source having a variable wavelength and intensity of light source. The eye's response to the wavelength and intensity of the light source may induce iris restriction to form a small pupil, and thereby a large iris. This process may allow for a diagnostic and/or treatment systems to more accurately set and maintain alignment and track eye movements during a procedure. By monitoring the affected changes in pupil and iris size, embodiments of the present system allow for better alignment of eyes and even eye movements using iris features before and during treatment. Affecting changes in pupil size is particularly useful as it increases the robustness of torsional tracking systems and aids in ensuring accurate alignment of an ablation pattern during a laser eye surgical procedure.

## Photopic and Scotopic Examples

The human eye reacts to differing light conditions by changing the size of its iris and thereby the pupil it forms. The iris acts like a muscle, constricting in bright light conditions in order to restrict how much light enters the eye and is received by the retina and relaxing in low light conditions in order to allow more light into the eye to be received by the retina.

Terms used to define various light conditions include Scotopic which may refer to conditions of dim light. Such a condition may be at a luminance level less than 3 cd/m$^2$. Scotopic vision occurs under luminance levels of $10^{-3}$ to $10^{-6}$ cd/m$^2$. Where cd is a candela. In such conditions, the iris would relax to reveal less iris and the pupil would be larger. Another term is Photopic which may refer to conditions of bright light. In such conditions, the iris would constrict to reveal more iris and the pupil would be smaller. Photopic light conditions may refer to daytime vision with luminance level 10 to $10^8$ cd/m$^2$. Mesopic may refer to conditions of low light but more light than Scotopic light, light levels ranging from luminance of approximately 0.001 to 3 cd/m$^2$.

Current iris registration (IR) methodology uses a scotopic eye image from the diagnostic device as a reference to register the photopic eye image from the laser system. However, iris distortions caused by pupil dilates and constricts may cause IR failure due to lack of matching blocks identified by the production algorithm from the two images. During each wavefront exam, diagnostic device captures a pair of images of the eye illuminated by a combination of infra-red and near infra-red light emitting diodes (LEDs) for scotopic illumination and a pair of green LEDs for scotopic illumination. The scotopic image was captured first, then the green LEDs were turned on and the photopic image was captured. This report compares results from image processing and IR using scotopic eye images and photopic eye images, and a new IR method is proposed to improve the IR capture rate.

A method which can be performed with the system of the present embodiments include obtaining a first image of the eye with an imaging device using scotopic conditions, obtaining a second image of the eye using photopic conditions, analyzing the iris and pupil in the first and second images, and utilizing the two images for alignment purposes when the patient undergoes laser procedure. The method may include centering and aligning a laser treatment with one or both of the images of the eye before and during performance of the laser treatment on the eye. The laser treatment may further include tracking positional movement and torsional rotation of the patient's eyes during a laser surgery procedure using the first and second images. A pupil center and a cyclotorsional angle (CT) of the eye is also determined to be used in the comparison of pupil center shift and CT angle offset.

The change in pupil size and iris feature may comprise a change of one millimeter or more. Because the pupil tracker on the laser system requires a pupil to be 6mm or less, but the wavefront aberrometer diagnostic tool requires a pupil of 6mm or more.

The change in pupil size may be calculated from optical information obtained by an optical sensor. The optical information may comprise a plurality of images obtained by an imaging device or other optical information.

A method which can be performed with the described system includes determining a light output to obtain a desired pupillary response to affect a change in pupil size, and directing the desired optical light output to the eye from a variable illumination source.

The current system may be incorporated into a laser eye surgical procedure having an eye tracking system. The tracking systems may comprise a torsional tracking systems that tracks cyclotorsional rotation. The tracking system may be configured to track a reference point of the eye, such as a pupil center location and an iris feature, by registering the reference point in images of the eye.

**A comparison of IR capture rate**

The table below gives the IR capture rate with 322 scotopic eye images and the 322 photopic eye images. The IR capture rate from the 322 scotopic eye images is 86.96%, while the IR capture rate from the 322 photopic eye images is 92.88%. The table IR capture rate with the 322 scotopic and photopic eve images.

|  | Image | IR Pass | Circular fitting pass | Elliptical fitting pass | IR pass rate % |
|---|---|---|---|---|---|
| Scotopic image | 322 | 280 | 268 | 12 | 86.96 |
| Photopic image | 322 | 300 | 297 | 3 | 92.88 |

Compared to the IR capture rate made by the scotopic eye images, the IR capture rate of the 322 photopic eye images increases 5.92%.

Since many laser eye surgery systems align to the pupil center, changes in location of the center of the pupil may cause misalignment of the ablation pattern. Additionally, torsional tracking systems typically align to markers on the eye, which may include the pupil center location and/or iris features. Loss of sufficient association between selected markers in iris and tracking images may result in loss of torsional tracking as the iris narrows, potentially leading to decentration of the ablation pattern and suboptimal treatment results. Examples of suboptimal results include astigmatism, halos, starbursts and decreased contrast sensitivity and decreased visual acuity in a patient's vision.

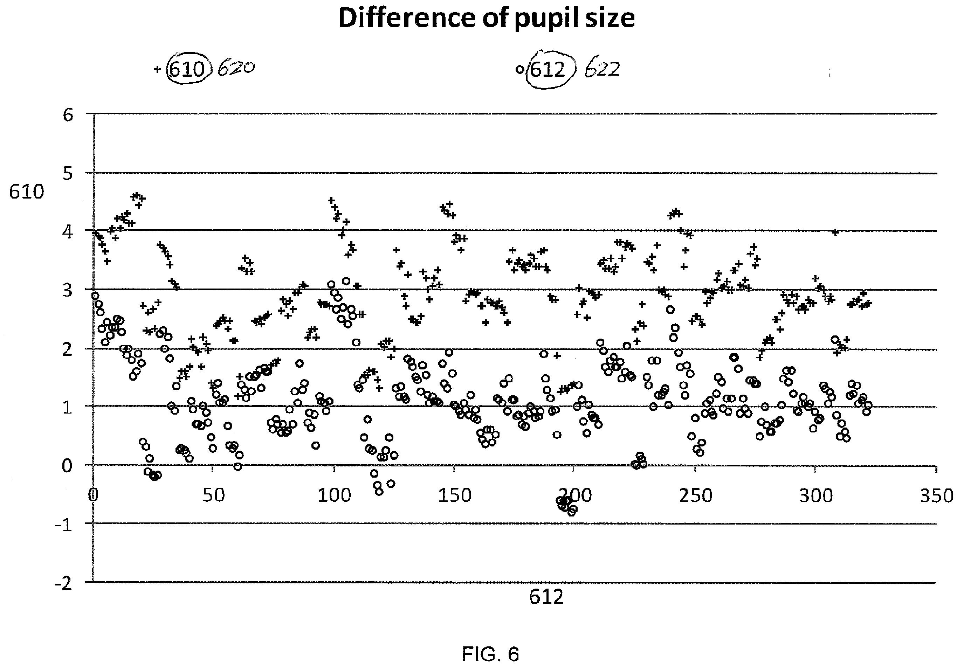

As the pupil changes due to adjustments of mesopic to photopic lighting conditions, the pupil center often drifts, as shown by the average pupil center shifts shown in FIG. 6. Generally as pupil size decreases, the pupil center will shift nasally, or toward the patient's nose. As pupil sizes increase, the pupil center shifts temporally, or toward the patient's temple. Significant changes in pupil size can occur quite rapidly and are often difficult to compensate for, especially when using a torsional tracking system. For example, if the pupil dilates significantly while its movement is being tracked with a torsional tracking system that detects eye movements based on pupil center location and/or iris features, the system may repeat a full registration to locate the shifted pupil center location or iris features of the dilated eye. Since full registration typically takes several seconds to complete, repeating full registration every time the pupil changes size is not desirable during a procedure. The claimed system, therefore, is advantageous as it affects the change in pupil size observed during surgery, thereby reducing pupil center drift allowing for improved torsional tracking and ablation centration. In particular, incorporating the disclosed system into any number of laser eye systems improves the robustness of tracking algorithms, and in particular facilitates robust torsional tracking.

The level at which changes in pupil size are changed can optionally be adjusted intermittently or continuously during a given diagnostic or treatment procedure. For instance, some procedures may be responsive to a threshold, relative to a target pupil size, so as to affect changes in pupil size when the pupil exceeds that threshold. For example, the threshold may range from about 1% to about 25% of the target pupil size. The threshold may be about 10% of the target size or the original pupil size. Referring now to FIG. **1,** system **10** includes a pupilometer **40,** an illumination source **30** having a variable optical light output **32,** and a processor **20**. The variable illumination source **30** and pupilometer **40** are coupled to processor **20**. Pupilometer **40** includes an optical sensor **42** and a processor **44** for determining changes in pupil size from optical information obtained by the optical sensor **42**. The optical sensor **42** is coupled to the eye E by an optical path **16**. Optical path **16** will often include additional optical imaging elements which are omitted from the simplified schematic of FIG. **1,** including imaging lenses and the like, so as to image an iris I of eye E onto an image sensing surface of imaging device **42**. Additional imaging components, such as apertures, filters, beam splitters, and the like can be used at least in part to define optical path **16,** and the optical components will typically be held in place by an appropriate metallic or polymer support structure, which may be integrated into a housing extending from an eye cup adjacent to the eye E and/or beyond imaging device **42**. Once the pupilometer **40** determines a change in pupil size, the pupilometer sends pupil size signals to processor **20**. Processor **20** then determines a desired optical light output to induce a desired pupillary response. The processor **20** then sends command signals to illumination source **30** to direct the desired optical light output **32** to the eye, thereby inducing a pupillary response. In some examples, system **10** rapidly repeats this process, continually affecting changes in pupil size to prevent significant changes in pupil size during the procedure.

Pupilometer **40** comprises or otherwise makes use of an optical sensor **42** coupled to a processor **44**. The processor **44** of the pupilometer **40** can be included within processor **20** or can be a separate processor. Optical sensor **42** comprises an image capture device, a camera, or any optical sensor capable of detecting optical information sufficient for determining changes in pupil size. The change in pupil size can be derived from measurement of the entire pupil P or may include only a part of the pupil P and iris I or iris boundary B. In some examples, the change in pupil size can be determined without directly measuring the size of the pupil P, for instance by registering multiple images of the pupil P or obtaining optical information from the pupil P and/or iris I as the pupil P changes in size. Typically, changes in pupil size are determined from comparing or registering multiple images of the eye E. However, optical information obtained from a sensor can be used to determine changes in pupil size without obtaining images, for instance optical changes at discrete points on the eye can indicate changes in the border of the pupil P. In an exemplary embodiment, the imaging device of the pupilometer **40** will obtain at least two images of the pupil, preferably a plurality of images in succession, so as to allow the pupilometer to calculate a change in the size of the pupil P relative to the first image or to a target pupil size. Changes in pupil size can also be expressed as a percentage by which the size of the pupil increases or decreases relative to the target pupil size.

In an exemplary embodiment, optical sensor **42** comprises a charge couple device ("CCD") which is sensitive to infrared light. Under infrared illumination, the pupil P of eye E will appear relatively dark as the infrared energy is not directly reflected by the clear corneal structure. The iris I surrounding the pupil P will present a much lighter shade to imaging device **42,** with the white scleral tissue surrounding the iris presenting a still lighter shade. The relatively high contrast borders between the pupil and iris and between the outer iris boundary and the surrounding tissues have a sufficiently high contrast image for determining pupil and iris size. Image processing software for use in determining the size and central location of pupil P and outer iris boundary B is commercially available from a number of sources. A variety of image processing software packages may be used, including (for example) *INTEL IMAGE* processing libraries or the like. Processors suitable for pupilometer **40** include PCs having at least the power of an *INTEL Pentium* processor. Many of the processors could also be used, including those running the MacOS operating system from *APPLE COMPUTERS,* INC., a custom DSP device, or the like. Alternative embodiments may make use of software modified from that of a commercially available pupilometer, such as the P2000 line of pupilometers sold by *PROCYON* of the United Kingdom.

Optical sensor **42** may comprise a wide variety of alternative image capture structures, including complementary metaloxide semiconductor ("CMOS") image capture devices, HRDC image capture devices, and the like. Optical sensor **42** may comprise, for example, a GW-902H model imaging device commercialized by GENWAC, INC. of New York and manufactured by W$_{ATEC}$ Co., LTD. of Japan, which may take images using IR illumination with a wavelength of 880 nm. A variety of alternative imaging devices, imaging structures, or other sensors might also be used, including a *GW-902B* model imaging device from GENWAC; a *Teli CE* imaging device which may take images using IR illumination with a wavelength of 940 nm, and/or another imaging device selected from those commercialized as the *CS8300B* series by TOKYO ELECTRONIC INDUSTRY CO., LTD of Japan; a 4900 model series imaging device commercialized by COHU, INC., Electronics Division of San Diego; and the like. Optical path **16** will typically image a field of view of at least about 10.5 mm by 14.0 mm (measured at the plane of the iris of the eye), onto the image sensing surface, so as to image a sufficient portion of the iris with the imaging device.

Variable illumination source **30** may comprise one or more light sources in optical communication with eye E. Illumination source **30** will typically have at least two settings of illumination levels, preferably having a plurality of illumination levels to create scotopic, mesopic, and photopic viewing conditions at the location of the eye E. The variable illumination source **30** can emit a constant optical light output or can emit a variable optical light output. An illumination source **30** may emit a variable optical light output by varying illumination from any or all of the sources or by altering the configuration, activating or deactivating light sources. An illumination source **30** comprising multiple light sources may include light sources having a constant illumination level, such as a light-emitting diode (LED). When system **10** is in use, illumination source **30** will illuminate the eye E with light having a wavelength suitable for inducing a pupillary response of the eye E. The optical light output from illumination source **30** may be directed to eye E by any manner sufficient to induce a pupillary response, including but not limited to ambient light, a halogen ring illuminator positioned around the eye, halogen oblique lights, a fixation light, an illuminated viewing target or any combination thereof.

In an exemplary embodiment, the intensity of the optical light output **32** from the variable illumination source **30** will be controlled by processor **20** using command signals sent to the illumination source **30.** During operation of system **10,** optical signals will typically be generated by optical sensor **42** and transmitted to processor **44** (which can be incorporated into processor **20**). Processor **44** will determine a change in the size of the pupil P, if any. In response to a determination of pupil size change, processor **44** will generate pupil size signals. The pupil size signals need not necessarily communicate the size of the pupils, but may include signals containing optical information from which the change in pupil size can be determined. In response to the pupil size signals from processor **44,** processor **20** determines a desired optical light output sufficient to induce a desired pupillary response to mitigate the change in pupil size. Processor **20** will then send a command signal to illumination source **30** to direct the desired optical light output **32** to eye E.

Processor **20** of system **10** will often comprise a computer **22,** as illustrated in FIG. **1.** In some embodiments, processor **20** will include a display **22** for showing an image of the structures of the eye, graphical representations of the size of the pupil, pupil drift or any other physiological characteristic measurements, and the like. Processor **20** will typically include a tangible media **29** embodying a machine readable code with programming instructions and/or data for implementing the method steps described herein. Tangible media **29** may comprise a magnetic recording media such as a floppy disk or magnetic tape, an optical recording media such as a CD or a DVD, an electronic media or memory such as a RAM or ROM, a nonvolatile memory such as a USB memory stick device, or the like. In some embodiments, the machine readable code and/or data may be transmitted via an Internet, an intranet, a wireless transmission device, an optical network or cable, an electrical coaxial or twisted pair cable, or the like. Alternative processor structures might also be used, including specialized processor boards, distributed data software and/or hardware arrangements, and the like. When in the form of a personal computer, processor **20** will typically include user input devices such as a keyboard and/or mouse, input and output ports, software such as an operating system and a pupilometer user interface. In some embodiments, the physician may input data, which may include a target pupil size, upper and lower limits of a range of target pupil sizes, and upper and lower limits of a range of acceptable optical light outputs.

System **10** may operate to change pupil size so as to direct the pupil size to a pre-determined range of sizes or within a tolerance of a target pupil size. For instance, system **10** may operate to affect changes in pupil size that exceed $\pm 10\%$ of a target pupil size. The target size of the pupil may be the original size of the pupil, the size of the pupil during the mapping of ocular aberrations or a pupil size as defined by a physician. System **10** may perform the described processes in iterations performed in quick succession. As in a feedback mechanism, once a detected change in pupil size has been made the system continues to detect and make subsequent changes in pupil size relative to the target pupil size. The system **10** may mitigate changes in pupil size in discrete time periods. For instance, the system may affect pupil sizes once every second, multiple times per second, or the timing of the process may incorporate a lag to account for the pupillary response lag to changes in optical output. The system **10** may use a time weighted average of pupil size changes to calculate a change in pupil size from which to determine the desired optical light output level. Alternatively, the system **10** may determine and affect pupil size changes dynamically as the pupillary response is induced.

System **10** may determine the desired optical light output **32** based on a relationship or trend of pupil size change for a given procedure. The relationship may be based on optical data gathered during the procedure, an average of optical data from multiple patients, a predictive algorithm, or information gathered during characterization of a patient's pupillary response. For instance, the processor **20** may calculate a range of pupil sizes and incorporate this into the determination of a desired illumination level. The processor **20** may also utilize a database of average pupillary responses to determine the desired optical light output. In some aspects, the processor **20** may calculate a relationship between a patient's pupil size and photopic and scotopic light conditions, in effect characterizing an eye E of a patient to predict pupillary responses. Calculating such relationships may be useful in determining desired light levels as pupillary responses may differ from patient to patient. For instance, an eye of a particular patient may be more sensitive to subtle changes in illumination than that of the average patient. By incorporating these relationships into the processor's determination of the desired optical light output, the processor **20** may increase the accuracy of the system **10** in affecting changes in pupil size.

FIG. **1**A schematically illustrates a simplified system of an embodiment of the present embodiments in a diagnostic system **100** and a treatment system **200**, which may optionally be integrated into a diagnostic/treatment system. In this embodiment, the diagnostic system is a wavefront measurement system **100.** The diagnostic procedure may be performed at the same time as the treatment procedure, or it may precede the treatment procedure by minutes, hours, days or weeks. The measurement system **100** is capable of generating images of the eye E and of providing information helpful for determining a desired corneal flap geometry. The flap geometry will often be referenced to the image, so that a relationship between the location of the flap incision and the image data can be established. The corneal flap geometry is often linked to a feature or reference location on the eye E which can be identified in the image, such as a pupil center (located at the center of the inner iris boundary), the center of the outer iris boundary or limbus, natural markings included in the iris, visible limbal landmarks or features, and the like. Along with locating and/or determining the desired corneal flap geometry, the measurement system **100** may also include at least a portion of a processor system capable of calculating a set of treatment instructions to be used by a laser incision system, such as femtosecond laser system **200**.

The exemplary measurement system **100** includes a wavefront measurement device **80**, such as a wavefront aberrometer, variable light source **30**, computer system **22,** and an imaging assembly **40**. Imaging assembly **40** captures an image of the eye at substantially the same time (so that the eye does not move between the image and the measurement) that the wavefront measurement device **80** directs a beam toward the eye of a patient in a diagnostic procedure under the direction of computer system **22**. Measurement device **80** and imaging assembly **40** may be optically coupled to optics **16,** which directs a measurement beam to the eye E, an image from the eye to the imaging assembly **40**, and a measurement image from the eye back to the measurement device. Variable light source **30** may be optical coupled to optics **16** or may be optically coupled to the eye E directly or through another path independent of optics **16**. The computer system **22** optionally determines a desired corneal flap geometry based on the images generated by the imaging assembly **40,** often with the input of a system operator. The computer may store the corneal flap geometry, wavefront measurements and images of the patient's eye. One or more different incisions, a set of incisions or the like may be calculated for a desired corneal flap geometry. While the incisions are generally applied to form the desired corneal flap geometry, an individual incision or set of incisions may optionally be calculated to be formed in the cornea. The computer system **22** also calculates a change in pupil size from the images obtained by imaging assembly **40**. From the calculated change in pupil size, computer system **22** determines a desired optical output sufficient to affect the change in pupil size according to predefined limits or user-defined variable. Computer system **22** then sends a control signal to variable light source **30** which directs the desired optical light output to the eye E so as to induce a pupillary response to affect the calculated change in pupil size so as to gather images of both larger and smaller pupil sizes and resulting irises. As the wavefront measurement and image are substantially contemporaneous, and as the structures of the imaging assembly and the measurement device are optically and/or mechanically coupled, the location information included in the image and the measurement can be associated. Computer system **22** may use the images of the eye E to contemporaneously direct measurement by the wavefront measurement device **80** and induce pupillary responses to affect changes in pupil size by the variable light source **30** during the measurement procedure. The computer processor **22** may also generate and save additional treatment information, such as an ablation profile or laser sculpting based on the image data that can later be downloaded into a refractive laser system **110** (see FIG. **1**B). Suitable measurement systems may include structures based on the *WaveScan Wavefront* System commercially available from Abbott Medical Optics, Inc. (AMO) of Santa Ana, Calif., the *Zyoptix* diagnostic workstation commercially available from Bausch and Lomb of Rochester, N.Y., and others.

The laser system **200** includes a laser **55,** such as a femtosecond laser, an imaging assembly **40** that obtains an image of the eye, a computer system **22** and a variable light source **30**. Images from imaging assembly **40** are used by computer system **22** to align an ablation profile to the cornea and may be further used by computer system **22** to track positional and/or rotational movements of the eye by registering features of the eye E. The computer system **22** also calculates a change in pupil size from the images obtained by imaging assembly **40**. In order to obtain an iris image that is suitable for alignment, computer system **22** sends a control signal to variable light source **30** which directs the desired optical light output to the eye E so as to induce a pupillary response to affect the calculated change in pupil size. Images from imaging assembly **40** are substantially contemporaneous with the incision of the eye using laser **55** and with adjustment of the variable light source **30** by the computer system **22**. The imaging assembly **40** and laser **55** are mechanically and/or optically coupled together, so that the images from imaging assembly **60** can be used by computer system **22** to help direct a laser beam **55** to the eye E of the patient during a treatment procedure. Treatment of the eye E with laser **55,** the computer system **22** adjusts variable light source **30** to induce a pupillary response so as to affect changes in pupil size during the procedure, thereby reducing changes in the tracked features of the eye relative to the cornea. Laser **55** and imaging assembly **60** may be optically coupled to optics **75,** which directs beam **65** to the eye E. Variable light source **30** is also coupled to eye E, either directly or indirectly, for instance through optics **16.** Computer system **22** will generally direct pulses of laser energy toward the cornea to form an incision in the cornea so as to form a flap of corneal tissue exposing the stroma underlying the corneal epithelium. Subsequent ablation or removal of the exposed stroma can alter the refractive characteristics of the eye. In some embodiments, the ablation profile generated with the measurement system **100** will be downloaded into computer system **22,** and the corneal correction may be performed using the femtosecond laser **55.** Suitable femtosecond laser systems may include the *iFS* Advanced Femtosecond Laser system commercially available from AMO.

The disclosed system can be readily adapted for use with existing laser systems, wavefront measurement systems, and other optical measurement devices. Although the systems, software, and methods of the present disclosure are described primarily in the context of a laser eye surgery system, it should be understood they may be adapted for use in alternative eye treatment procedures, systems, or modalities, such as spectacle lenses, intraocular lenses, accommodating IOLs, contact lenses, corneal ring implants, collagenous corneal tissue thermal remodeling, corneal inlays, corneal onlays, other corneal implants or grafts, and the like. Relatedly, systems, software, and methods disclosed herein are well suited for customizing any of these treatment modalities to a specific patient. Thus, for example, examples encompass custom intraocular lenses, custom contact lenses, custom corneal implants, and the like, which can be configured to treat or ameliorate any of a variety of vision conditions in a particular patient based on their unique ocular characteristics or anatomy. Aspects of techniques described herein can be implemented in a variety of laser and aberrometer devices, including without limitation the *iDesign Advanced WaveScan Studio* System, *WaveScan WaveFront* System and *STAR S4 IR* Excimer Laser System, the *Wavelight Alegretto* System and the Tscherning-based aberrometer; the Alcon *Ladarvision* lasers and *Ladarwave* aberrometer; the Bausch and Lomb *Zyoptix* laser and related aberrometer, and the Zeiss laser and **WASCA** aberrometer.

Turning now to the drawings, FIG. **1**B illustrates a laser eye surgery system **210** of the present embodiments, including a laser **55** that produces a laser beam. Laser **55** is optically coupled to laser delivery optics **16,** which directs laser beam to an eye E of patient P. A delivery optics support structure (not shown here for clarity) extends from a frame **18** supporting laser **55.** A microscope **70** is mounted on the delivery optics support structure. A pupilometer **40** is positioned so as to measure the size of the pupil of the eye. In some embodiments the pupilometer **40** will be coupled to microscope **70** which will be used to image a cornea of eye E. Laser **55** generally comprises an excimer laser, comprising an argonfluorine laser producing pulses of laser light having a wavelength of approximately 193 nm. Laser **55** will preferably be designed to provide a feedback stabilized fluence at the patient's eye, delivered via delivery optics **16.** The present embodiments may also be useful with alternative sources of ultraviolet or infrared radiation, particularly those adapted to controllably ablate the corneal tissue without causing significant damage to adjacent and/or underlying tissues of the eye. Such sources include, but are not limited to, solid state lasers and other devices which can generate energy in the ultraviolet wavelength between about 185 and 205 nm and/or those which utilize frequency multiplying techniques. Hence, although an excimer laser is the illustrative source of an ablating beam, other lasers may be used.

Laser system **210** will generally include a computer **22** or programmable processor **20** coupled to the pupilometer **40** or other imaging device, the laser **55** and the variable illumination source **30**. Processor **20** may comprise (or interface with) a conventional PC system including the standard user interface devices such as a keyboard, a display monitor, and the like. Processor **20** will typically include an input device such as a magnetic or optical disk drive, an internet connection, or the like. Such input devices will often be used to download a computer executable code from a tangible storage media **29** embodying any of the disclosed methods Tangible storage media **29** may take the form of a floppy disk, an optical disk, a data tape, a volatile or nonvolatile memory, RAM, or the like, and the processor **20** will include the memory boards and other standard components of modern computer systems for storing and executing this code. Tangible storage media **29** may optionally embody wavefront sensor data, wavefront gradients, a wavefront elevation map, a treatment map, a corneal elevation map, and/or an ablation table. While tangible storage media **29** will often be used directly in cooperation with an input device of processor **20,** the storage media may also be remotely operatively coupled with processor by means of network connections such as the internet, and by wireless methods such as infrared, Bluetooth, or the like.

Laser **55** and delivery optics **16** will generally direct laser beam to the eye of patient P under the direction of a computer **22** during which variable light source **30** emits light to the eye along optical path of delivery optics **16.** Computer **22** will often selectively adjust laser beam contemporaneous with adjusting the output of the variable illumination source **40.** The computer **22** adjusts the laser beam to expose portions of the cornea to the pulses of laser energy so as to effect a pre-determined sculpting of the cornea and alter the refractive characteristics of the eye. Contemporaneous with the laser beam treatment, the computer **22** may selectively adjust the variable light source **30** to direct an optical output to the eye sufficient to affect the calculated changes in pupil size (as calculated from images obtained from pupilometer **40**). The computer **22** may also adjust the variable light source **30** to effect a pre-determined change in pupil size if desired. In many embodiments, the laser beam, the laser delivery optical system **16,** and the variable light source **30** will be under computer control of processor **22** to effect the desired laser sculpting process, with the processor effecting (and optionally modifying) the pattern of laser pulses and the light output of the variable light source. The desired light outputs may by summarized in machine readable data of tangible storage media **29** in the form of a treatment table, and the treatment table may be adjusted according to feedback input into processor **22** from an automated image analysis system or pupilometer **40** in response to feedback data provided from an ablation monitoring system feedback system. Optionally, the feedback may be manually entered into the processor by a system operator. Such feedback might be provided by integrating the wavefront measurement system described below with the laser treatment system **10,** and processor **22** may continue and/or terminate a sculpting treatment in response to the feedback, and may optionally also modify the planned sculpting based at least in part on the feedback. Measurement systems are further described in U.S. Pat. No. 6,315,413.

Optical light source **30** may be adjusted to produce the desired pupillary response using a variety of alternative mechanisms. The light source **30** may be selectively limited using one or more variable apertures. An exemplary variable aperture system having a variable iris and a variable width slit is described in U.S. Pat. No. 5,713,892. Typically, halogen ring illuminators and halogen oblique lights
can be adjusted by discrete steps, such as in a dimmer switch. The fixation light source, where the patient is instructed to look during surgery, can also be made adjustable. In some embodiments, the source may include an existing illumination source in a diagnostic or treatment device, such as the visible light source in the *iDesign Advanced WaveScan Studio* System. Other illumination sources, including those based on LEDs, which may be used as alternative or additional sources of visible illumination of the eye during surgery. Using a plurality of light sources, as described above, allows for high dynamic range of illumination and further allows for changes of light control to change pupil size and/or provide a well illuminated surgical area for the operating physician.

Additional components and subsystems may be included with laser system 210, as should be understood by those of skill in the art. For example, spatial and/or temporal integrators may be included to control the distribution of energy within the laser beam, as described in U.S. Pat. No. 5,646,791. Ablation effluent

evacuators/filters, aspirators, and other ancillary components of the laser surgery system are known in the art. Further details of suitable systems for performing a laser ablation procedure can be found in commonly assigned U.S. Pat. Nos. 4,665,913, 4,669,466, 4,732,148, 4,770,172, 4,773,414, 5,207,668, 5,108,388, 5,219,343, 5,646,791 and 5,163,934. Suitable systems also include commercially
available refractive laser systems such as those manufactured and/or sold by Alcon, Bausch & Lomb, Nidek, Wave-Light, LaserSight, Schwind, Carl Zeiss Meditec, and the like. Basis data can be further characterized for particular lasers or operating conditions, by taking into account localized environmental variables such as temperature, humidity, airflow, and aspiration.

FIG. **1**C is a simplified block diagram of an exemplary computer system **22** that may be used by the laser surgical system **10** of the present embodiments. Computer system **22** typically includes at least one processor **52** which may communicate with a number of peripheral devices via a bus subsystem **54.** These peripheral devices may include a storage subsystem **56,** comprising a memory subsystem **58** and a file storage subsystem **60,** user interface input devices **62,** user interface output devices **64,** and a network interface subsystem **66.** Network interface subsystem **66** provides an interface to outside networks **68** and/or other devices, such as a wavefront measurement system.

User interface input devices **62** may include a keyboard, pointing devices such as a mouse, trackball, touch pad, or graphics tablet, a scanner, foot pedals, a joystick, a touchscreen incorporated into the display, audio input devices such as voice recognition systems, microphones, and other types of input devices. User input devices **62** will often be used to download a computer executable code from a tangible storage media **29** embodying any of the disclosed methods. In general, use of the term *input device* is intended to include a variety of conventional and proprietary devices and ways to input information into computer system **22.**

User interface output devices **64** may include a display subsystem, a printer, a fax machine, or nonvisual displays such as audio output devices. The display subsystem may be a cathode ray tube (CRT), a flat panel device such as a liquid crystal display (LCD), a projection device, or the like. The display subsystem may also provide a nonvisual display such as via audio output devices. In general, use of the term *output device* is intended to include a variety of conventional and proprietary devices and ways to output information from computer system **22** to a user.

Storage subsystem **56** can store the basic programming and data constructs that provide the functionality of the various embodiments. For example, a database and modules implementing the functionality of any of the disclosed methods as described herein, may be stored in storage subsystem **56.** These software modules are generally executed by processor **52.** In a distributed environment, the software modules may be stored on a plurality of computer systems and executed by processors of the plurality of computer systems. Storage subsystem **56** typically comprises memory subsystem **58** and file storage subsystem **60.**

Memory subsystem **58** typically includes a number of memories including a main random access memory (RAM) **70** for storage of instructions and data during program execution and a read only memory (ROM) **72** in which fixed instructions are stored. File storage subsystem **60** provides persistent (non-volatile) storage for program and data files, and may include tangible storage media **29** (FIG. **1**B) which may optionally embody pupil size data, anticipated pupillary response tables, wavefront sensor data, wavefront gradients, a wavefront elevation map, a treatment map, and/or an ablation table. File storage subsystem **60** may include a hard disk drive, a floppy disk drive along with associated removable media, a Compact Digital Read Only Memory (CD-ROM) drive, an optical drive, DVD, CDR, CDRW, solid-state removable memory, and/or other removable media cartridges or disks. One or more of the drives may be located at remote locations on other connected computers at other sites coupled to computer system **22.** The modules implementing the functionality of embodiments may be stored by file storage subsystem **60.**

Bus subsystem **54** provides a mechanism for letting the various components and subsystems of computer system **22** communicate with each other as intended. The various subsystems and components of computer system **22** need not be at the same physical location but may be distributed at various locations within a distributed network. Although bus subsystem **54** is shown schematically as a single bus, alternate embodiments of the bus subsystem may utilize multiple busses.

Computer system **22** itself can be of varying types including a personal computer, a portable computer, a laptop computer, a workstation, a computer terminal, a network computer, a smartphone, a tablet computer, a control system in a wavefront measurement system or laser surgical system, a mainframe, or any other data processing system. Due to the ever-changing nature of computers and networks, the description of computer system **22** depicted in FIG. **1**C is intended only as a specific example for purposes of illustrating some embodiments. Many other configurations of computer system **22** are possible having more or less components than the computer system depicted in FIG. **1**C.

Referring now to FIG. **1**D, one embodiment of a laser eye wavefront system **110** is schematically illustrated in simplified form. More specifically, system **110** includes a wavefront sensor **80**, a variable light source **30**, such as an LED, which can be adjusted to direct a desired light output to the optical tissues of eye E so as to induce a pupillary response. In very general terms, measurement system **110** is configured to perform wavefront measurements and detect changes in pupil size during a procedure and adjust the variable optical light source in response to a calculated change in pupil size to affect a desired pupillary response. System **110** may be used in conjunction with laser system **210**. The wavefront sensor **80** communicates signals to a computer system **22'** for measurement of the optical errors in the optical tissues **34** and/or determination of an optical tissue ablation treatment program. Computer **22'** may include the same or similar hardware as the computer system **22** illustrated in FIGS. **1**B and **1**C. Computer system **22'** may be in communication with computer system **22** that directs laser surgery system **210,** or some or all of the components of computer system **22, 22'** of the wavefront measurement system **110** and laser surgery system **210** may be combined or separate. If desired, data from wavefront sensor **80** may be transmitted to a laser computer system **22** via tangible media **29,** via an I/O port, via a networking connection **66** such as an intranet or the Internet, or the like.

Wavefront sensor **80** generally comprises a lenslet array **86** and an image sensor **82**. As the image from retina R is transmitted through optical tissues **34** and imaged onto a surface of image sensor **82** and an image of the eye pupil P is similarly imaged onto a surface of lenslet array **886,** the lenslet array separates the transmitted image into an array of beamlets **84,** and (in combination with other optical components of the system) images the separated beamlets on the surface of sensor **82**. Sensor **82** typically comprises a charged couple device or CCD, and senses the characteristics of these individual beamlets, which can be used to determine the characteristics of an associated region of optical tissues **34**. In particular, where image **44** comprises a point or small spot of light, a location of the transmitted spot as imaged by a beamlet can directly indicate a local gradient of the associated region of optical tissue.

Eye E generally defines an anterior orientation ANT and a posterior orientation POS. Optical light source **30** or another projection source generally projects an image in a posterior orientation through optical tissues **34** onto retina R as indicated in FIG. **1**D. Optical tissues **34** again transmit image **44** from the retina anteriorly toward wavefront sensor **36**. Image **44** actually formed on retina R may be distorted by any imperfections in the eye's optical system when the image source is originally transmitted by optical tissues **34**. Eye E generally defines an anterior orientation ANT and a posterior orientation POS. An image is protected from optical light source **30** or another light source in optical communication with eye E. The image is projected in a posterior orientation through optical tissues **34** onto retina R as shown in FIG. **1**D.

In some embodiments, image source optics **46** may decrease lower order optical errors by compensating for spherical and/or cylindrical errors of optical tissues **34**. Higher order optical errors of the optical tissues may also be compensated through the use of an adaptive optic element, such as a deformable mirror (described below). Use of an image source **32** selected to define a point or small spot at image **44** upon retina R may facilitate the analysis of the data provided by wavefront sensor **80**. Distortion of image **44** may be limited by transmitting a source image through a central region **48** of optical tissues **34** which is smaller than a pupil **50**, as the central portion of the pupil may be less prone to optical errors than the peripheral portion. Regardless of the particular image source structure, it will be generally be beneficial to have a well-defined and accurately formed image **44** on retina R.

Optical light source **30** may be a light source or variable optical light source and is coupled with the process or computer **22'** such that the processor can send command signals to the optical light source to direct a desired optical output level of illumination to the eye. The optical light source **30** may be the same optical source that projects an image on the retina or may be separate, such as a halogen ring placed near the eye E during the procedure. Computer **22'** calculates changes in pupil size during the procedure from images obtained by the imagine assembly **40**. In response to a calculated change in pupil size, computer **22'** determines a desired optical output sufficient to induce a pupillary response, such that the pupillary response affects the change in pupil size to a desired state for imaging and alignment purposes. In response to the calculated change in pupil size, computer **22'** sends a command to optical light source **30** to direct the desired optical output to the eye E to induce the desired pupillary and iris response.

In one embodiment, the wavefront data may be stored in a computer readable medium **29** or a memory of the wavefront sensor system **30** in two separate arrays containing the x and y wavefront gradient values obtained from image spot analysis of the Hartmann-Shack sensor images, plus the x and y pupil center offsets from the nominal center of the Hartmann-Shack lenslet array, as measured by the pupil camera **40** (FIG. **1**D) image. Such information contains all the available information on the wavefront error of the eye and is sufficient to reconstruct the wavefront or any portion of it. In such embodiments, there is no need to reprocess the Hartmann-Shack image more than once, and the data space required to store the gradient array is not large. For example, to accommodate an image of a pupil with an 8 mm diameter, an array of a 20x20 size (i.e., 400 elements) is often sufficient. As can be appreciated, in other embodiments, the wavefront data may be stored in a memory of the wavefront sensor system in a single array or multiple arrays.

While many methods which can be performed with the system of the present embodiments will generally be described with reference to sensing of an image **44,** a series of wavefront sensor data readings may be taken. For example, a time series of wavefront data readings may help to provide a more accurate overall determination of the ocular tissue aberrations. As the ocular tissues can vary in shape over a brief period of time, a plurality of temporally separated wavefront sensor measurements can avoid relying on a single snapshot of the optical characteristics as the basis for a refractive correcting procedure. Still further alternatives are also available, including taking wavefront sensor data of the eye with the eye in differing configurations, positions, and/or orientations. For example, a patient will often help maintain alignment of the eye with wavefront measurement system **30** by focusing on a fixation target, as described in U.S. Pat. No. 6,004,313. By varying a position of the fixation target as described in that reference, optical characteristics of the eye may be determined while the eye accommodates or adapts to image a field of view at a varying distance and/or angles.

The location of the optical axis of the eye may be verified by reference to the data provided from an imaging device **40**. Imaging device **40** may also act as a pupilometer obtaining images of at least a portion of the pupil from which the computer **22'** can calculate changes in pupil size. In the exemplary embodiment, imaging assembly **40** images pupil **50** so as to determine a position of the pupil for registration of the wavefront sensor data relative to the optical tissues, for instance by registering pupil center location and/or iris features relative to the cornea. By monitoring changes in pupil size and affecting such changes through the variable optical output, the system may better image iris features relative to the cornea improving tracking and registration of the cornea during the procedure.

An embodiment of a wavefront measurement system **120** is illustrated in FIG. **IE.** The major components of the system of FIG. **IE** are similar to those of FIG. **1**D. Additionally, FIG. **IE** includes an adaptive optical element in the form of a deformable mirror. The source image is reflected from deformable mirror **98** during transmission to retina R, and the deformable mirror is also along the optical path used to form the transmitted image between retina **R** and imaging sensor **40.** Deformable mirror **98** can be controllably deformed by computer system **22** to limit distortion of the image formed on the retina or of subsequent images formed of the images formed on the retina, and may enhance the accuracy of the resultant wavefront data. The computer **22** calculates any changes in pupil size from images obtained from the CCD and determines the desired optical output and commands variable optical light source **30** to direct the desired optical output, according to the parameters of the system. The structure and use of the system of FIG. **IE** are more fully described in U.S. Pat. No. 6,095,651.

The components of an embodiment of a wavefront measurement system for measuring the eye and ablations may comprise elements of a WaveScan® system, available from VISX, INCORPORATED of Santa Clara, Calif. One embodiment includes a WaveScan system with a deformable mirror as described above. An alternate embodiment of a wavefront measuring system is described in U.S. Pat. No. 6,271,915. It is appreciated that any wavefront aberrometer could be employed for use with

embodiments here. Relatedly, some embodiments encompass the implementation of any of a variety of optical instruments provided by WaveFront Sciences, Inc., including the COAS wavefront aberrometer, the *ClearWave* contact lens aberrometer, the *CrystalWave* IOL aberrometer, and the like.

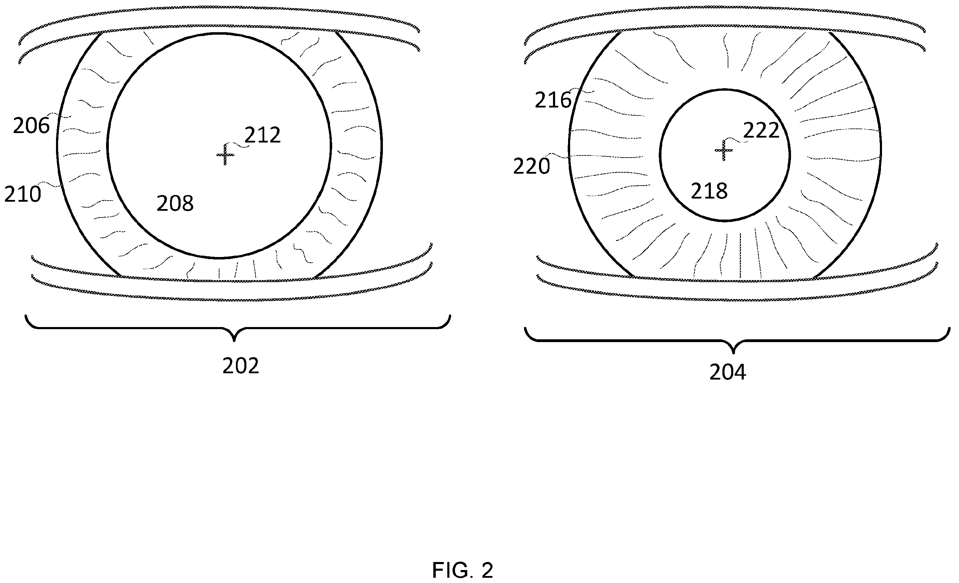

FIG. **2** generally shows a side by side image of an eye as obtained by camera **40** taken as a pupil is dilated **202** and the associated iris 206; and constricted **204** along with the associated iris 216; also shown are, pupil center locations **212, 222** of the dilated 202 and constricted 204 eyes. Camera **40** may generally comprise an image capture device or other optical sensor capable of detecting optical information sufficient for measurement of the pupil center location, **212, 222** pupil size **208, 218,** outer iris boundary size **210, 220** and/or location, the location of other additional or alternative reference structures on the eye, and the like.

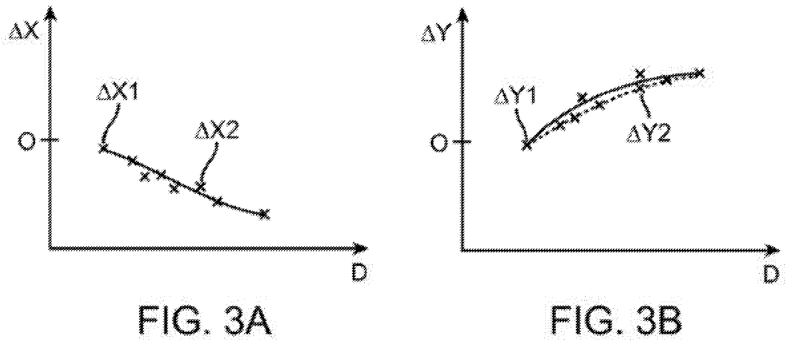

FIG. 3A and **3B** show charts of example pupil diameter on the x axis and in they axis, the change in the X direction for FIG. 3A and change in the Y direction in FIG. 3B.

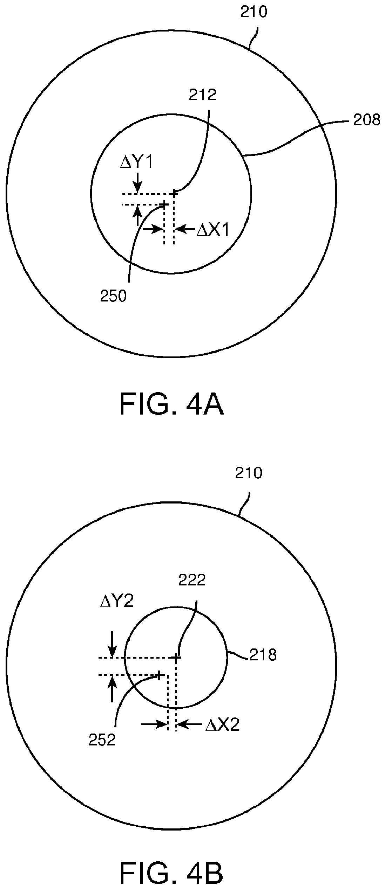

FIG. **4A** and **4B** show an eye with a dilated pupil 4A and constricted pupil **4B.** The FIGs reference to the outer iris boundary **210,** pupil boundary **208,** constricted pupil boundary **218,** center of pupil **212** and center of constricted pupil **222** as well as the center of the iris **250** and center of iris in constricted pupil eye **252.**

As illustrated in FIGs. **2, 3A, 3B,** and **4A,** a location of the iris **206, 216** (and all other tissues of the eye) will change with saccadic and other movements of the eye. Using the difference in relative contrast between the pupil **208** and surrounding iris **206,** pupilometer 40 determines a diameter of pupil **208** and identifies a center location **212.** Similarly, using the same image captured by camera **40,** processor **20** also determines a diameter of the outer iris boundary **210** and a location of the boundary center **250,** generally by using the contrast differential between the outer iris boundary and the surrounding tissues. Based on the difference in location between the outer iris boundary center 250 and the pupil center boundary 212, processor **20** identifies a horizontal center difference Δx and a vertical center difference Δy. FIG. 3A and FIG. **3B** shows a plot of the identified Δxl and Δyl locations as the pupil size changes.

Referring now to FIGS. **2,** 3A-3B and FIG. **4B,** a subsequent image taken shows the constricted smaller pupil size **218** after pupil constriction. Processor **20** once again determines a size and center location of the constricted pupil **222** relative to the concurrent outer iris boundary center **252** so as to determine new horizontal and vertical center offsets Δx2 and Δy2. By measuring a series of different viewing distances, horizontal and vertical pupil center drift with changing viewing distance D may be plotted as shown in FIG. **3A** and FIG. **3B.**

Image processing software for use in determining the size and central location of pupil **208** and outer iris boundary **210** is commercially available from a number of sources. A variety of image processing software packages may be used, including (for example) INTEL IMAGE processing libraries or the like. Many of the processors could also be used, including those running the MacOS operating system from APPLE COMPUTERS, INC., a custom DSP device, or the like. Alternative embodiments may make use of software modified from that of a commercially available pupilometer, such as the P2000 line of pupilometers sold by PROCYON of the United Kingdom.

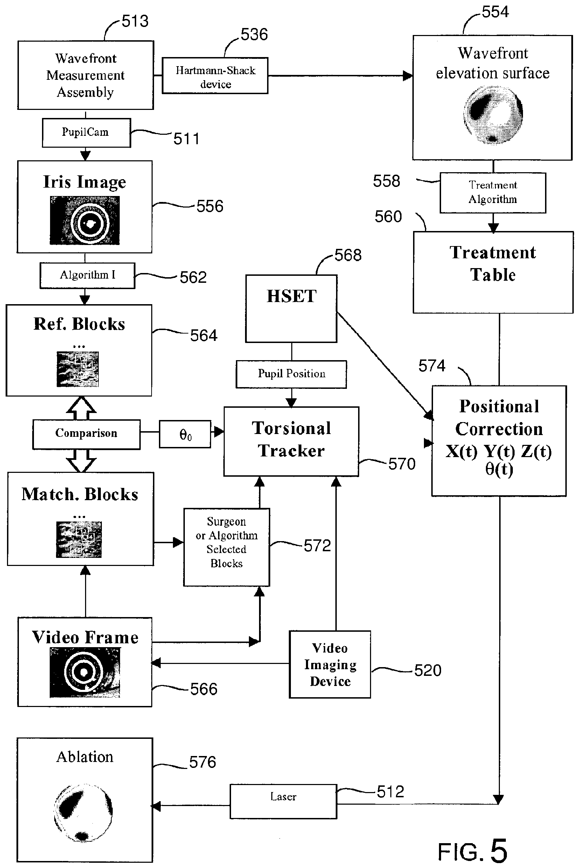

Referring now to FIG. **5,** an information flow of a method which can be performed with the system of the present disclosure is described. Wavefront measurement assembly **513** can use wavefront sensors **536,** such as Hartmann-Shack sensors, for obtaining a wavefront elevation surface **554** of the patient's eye. Wavefront elevation surface **554** can be run through a treatment algorithm **558** to generate a treatment table or ablation profile **560** that is customized to correspond to the patient's wavefront elevation surface **554**. As noted above, ablation profile **560** can be calculated by a processor of wavefront device **510,** laser system **515,** or by a separate processor and stored in a memory of computer **22.**

During the calculation of the wavefront elevation surface, pupil camera **511** can concurrently obtain an image **556** of the patient's eye, e.g., pupil and iris. The image of the patient's eye **556** can be analyzed by an algorithm **562** that locates the center of the pupil and/or iris, calculates the radius of the pupil and/or iris, and locates markers **564** in the patient's iris for subsequent registration and tracking.

In order to register the ablation profile **560** and the patient's eye during the laser treatment, the ablation pattern and the patient's eye should share a common coordinate system. Thus, ablation profile **560** should be positionally and torsionally aligned with the patient's eye when the patient's eye is positioned in the path of the laser beam. Additionally, the translational and torsional orientation of the patient's eye should be tracked during the surgical procedure to ensure an accurate delivery of the ablation profile.

To torsionally align (i.e., register) the ablation profile **560** with the patient's eye E, the reference or iris image **556** of the eye needs to have a unique coordinate transformation to an image of the eye taken by the pupil camera **511** of the laser system so as to determine the positional differences and torsional offset between the two images of the eye, $\theta_o$.

In exemplary embodiments, video imaging device **520** is a video device that can obtain streaming video of the patient's eye. One frame **566** of the streaming video, typically the first frame of the streaming video, can be analyzed by the computer processor to locate the pupil center, pupil size, iris center, and/or markers **564** that were originally located in the reference image **556**. Once the pupil center, iris center, and/or markers **564** are located, a torsional offset, $\theta_o$, between reference image **556** and video frame image **566** of the patient's eye may be calculated.

Once the torsional offset $\theta_o$ is determined, the computer can track the translational position (x(t), y(t), and z(t)) of the patient's eye E with a high speed eye tracker (HSET) **568** and the torsional orientation ($\theta$(t)) of the eye with a torsional tracker **570**. Because the position of the center of the pupil is tracked with the HSET **568,** the torsional tracker **570** generally estimates the position of the markers **564** with respect to the pupil center.

If the HSET **568** determines that the patient's eye has moved (relative to video frame image **566),** the computer can correct the delivery of the customized ablation pattern by adjusting the patient's customized treatment table **560** by adding in the translation and torsional measurements into the table. The treatment table can be adjusted such that at time t, if the overall rotation angle of the eye is $\theta$(t), and the next pulse of the laser is supposed to be delivered at location (x,y) on the cornea, the new location of the delivery of the pulse can be defined by:

$$\begin{pmatrix} x' \\ y' \end{pmatrix} = \begin{bmatrix} \cos\theta & -\sin\theta \\ \sin\theta & \cos\theta \end{bmatrix} \begin{pmatrix} x \\ y \end{pmatrix}$$

To track the torsional movement of the patient's eye, torsional tracker **570** can use the markers **564** identified above, other high-contrast iris patches, or if the patient's iris contains too little texture, the surgeon will have an option of drawing artificial landmarks **572** on the eye for tracking. Optionally, in some embodiments it is possible for the algorithm to decide if artificial markers are required.

The translational position and torsional orientation of the patient's eye can be tracked and analyzed by a computer processor in real-time so that the x(t), y(t), z(t) and 8(t) information **574** can be used to adjust the customized treatment table 560 so that laser **512** delivers the appropriate ablation pattern **576** to the patient's eye. Various examples of this torsional tracking system are described in U.S. Pat. No. 7,044,602.

FIG. **6** is an example chart showing pupil diameter differences **610** in millimeters on the y axis and eyes **612** on the x axis. The chart shows comparisons of pupils taken in scotopic environments **620** and photopic environments **622** as compared to pupils under the laser treatment environment. The chart shows that images of pupils taken under photopic conditions are more similar to images of pupils taken by the laser treatment system than images of pupils taken under scotopic conditions.

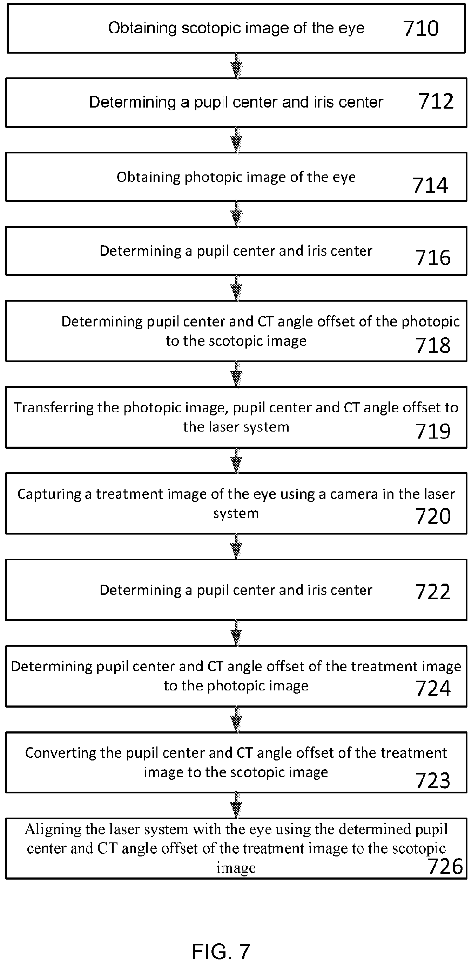

FIGS. **7-8,** schematically illustrate exemplary methods which can be performed with the system according to the invention. These methods do not form a part of the invention. In the example shown in FIG. **7,** the method can be performed with a laser eye surgery system. The method first obtains a scotopic image of the eye **710**. In some embodiments, this may be done with a Wavefront diagnostic system. Next, the method includes determining a pupil center and iris center **712**. Next, the method includes capturing a photopic image of an iris in the eye **714** and determining a pupil center and iris center **716**. Next the method includes determining, by the computer, the pupil center and CT angle offset of the photopic to the scotopic image of the iris **718** for transfer of the photopic image, pupil center, and CT angle offset to a laser system **719.** Next, the method includes capturing a treatment image of the eye using a camera in the laser system **720** and determining a pupil center and iris center **722.** Next, determining pupil center and CT angle offset of the treatment image to the photopic image **724.** Next, converting the pupil center and CT angle offset of the treatment image to the scotopic image **723.** Finally, aligning the laser system with the eye using the determined pupil center and CT angle offset of the treatment image to the scotopic image **726.** In some embodiments, the order of the steps in FIG. **7** could be accomplished in a different order, the listing in this order is not intended to be limiting.

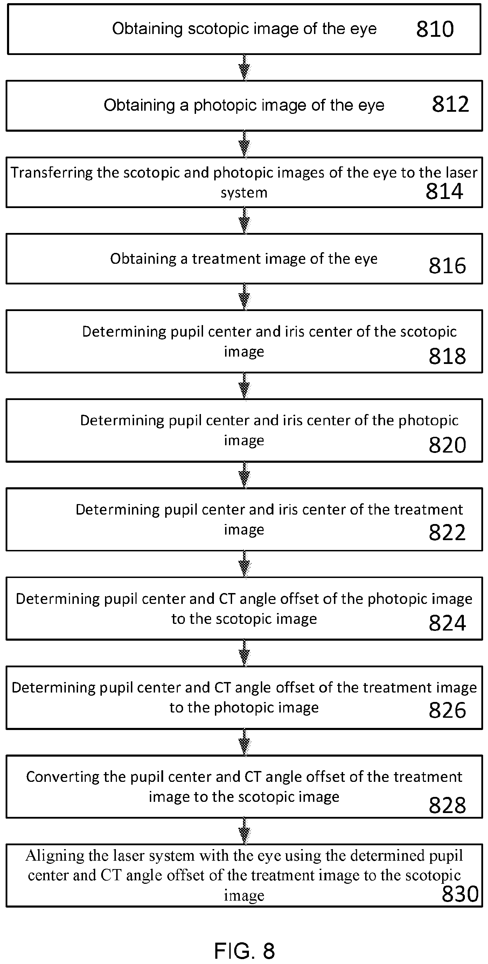

FIG. **8** shows an example method which can be performed with the inventive system. The first step of the method is to obtain a scotopic image of an eye **810.** Obtaining such an image may be through capturing an image with a camera system in a Wavefront diagnostic system as described herein. The next step is to obtain a photopic image of the eye **812.** Again, this could be by a Wavefront diagnostic system. The next step is transferring the scotopic and photopic images to the laser system **814.** The next step is to obtain an image of the eye using the treatment laser system **816.** The next step of the method includes determining the pupil center and iris center of the scotopic image **818.** The next step of the method includes determining the pupil center and iris center of the photopic image **820.** The next step of the method includes determining the pupil center and iris center of the treatment laser system image **822.** In some embodiments, the three preceding steps **818, 820** and **822** may be accomplished in any order or at the same time. The next step of the method includes determining a pupil center and CT angle offset of the photopic image to the scotopic image **824.** The next step of the method includes determining a pupil center and CT angle offset of the treatment image to the photopic image **826.** In some embodiments, the two previous steps **824** and **826** may be accomplished in any order or at the same time. The next step is converting the pupil center and CT angle offset of the treatment image to the scotopic image **828.** The final step in the method includes aligning the laser system with the eye using the determined pupil center and CT angle offset of the treatment image to the scotopic image **830.** In some embodiments, the order of the steps in FIG. **8** could be accomplished in a different order, the listing in this order is not intended to be limiting.

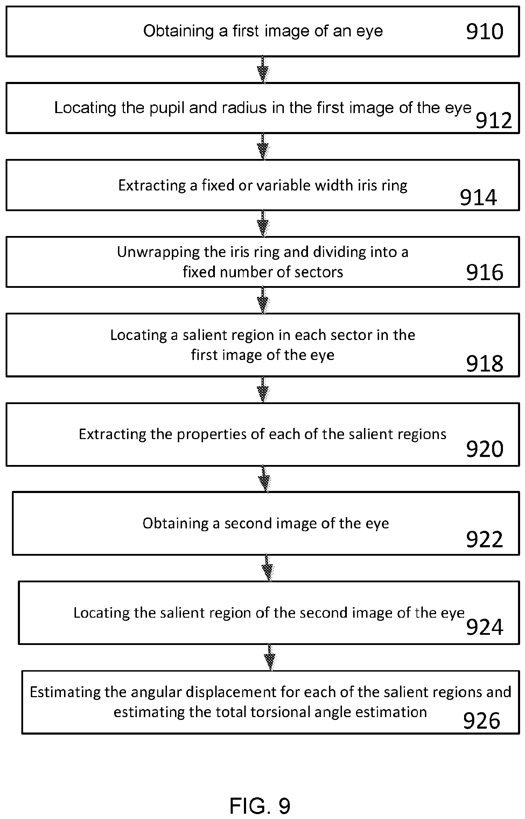

FIG. **9** shows an example method which does not form a part of the invention and illustrates the data flow through an alignment algorithm that can torsionally register a reference image with a second image of the eye to determine the torsional displacement between the two images of the eye. An initial step in the method **910** is to obtain the first, reference image. The first or reference image may be a grayscale image of the patient's eye that is taken by a CCD camera in the wavefront measurement device under infrared illumination (X=940 nm). In one test configuration, the images were 768x576 pixels and have 256 gray levels. The image contains the pupil and the iris. In some images, part of the iris may be occluded by one or both of the eyelids or cropped by the camera's field of view. It should be appreciated however, that a variety of imaging devices can be used to produce different images and can be illuminated under various types of illumination.

In most configurations, the smallest distance between the edge of the pupil and the obstructing elements, such as eyelids, eyelashes, strong shadows or highlights should be sufficiently large to leave a portion of the iris completely exposed for the entire 360-degree range. Preferably, the largest possible portion of the iris is in sharp focus so as to expose its texture. A pupil finding algorithm can be used to locate the pupil, calculate the radius of the pupil and find the center of the pupil **912.** The method continues with extracting a fixed or variable width iris ring **914.** Next, the system may continue by unwrapping the iris ring and dividing it into a fixed number of sectors **916.** Next, the system may continue the method by locating a salient region in each sector in the first image of the eye **918.** Next, the system may continue by extracting the properties of each of the salient regions **920.** Next, the system may continue by obtaining a second image of the eye **922.** Next, the system may continue by locating the salient region of the second image of the eye **924.** Finally, the system may complete the method by estimating the angular displacement for each of the salient regions and estimating the total torsional angle estimation **926.**

In some embodiments the pupil may be located by thresholding the image by analyzing a pixel value histogram and choosing the position of a first dip in the histogram after at least 2000 pixels are below the cutoff threshold. All pixels below the threshold are labeled with "1" and pixels above the threshold are labeled with "0". Pixels labeled with " 1" would generally correspond to the pupil, eyelashes, and possibly other regions of the image. It should be appreciated however, that the number of pixels employed will be related to the area of the pupil and will vary .

Optionally, in some embodiments an iris finding algorithm can be used to locate the iris, calculate the radius of the iris, and/or locate the iris center. Since the images of the eye from both imaging assembly and the camera both contain the pupil and iris, in some embodiments it may be more accurate to register the images by calculating the center of the pupil and the center of the iris and expressing the position of the pupil center with respect to the center of the iris. The center of the iris may be described as a center of a circle corresponding to the outer boundary of the iris. The position of the center of the iris can be used to calculate a pupil offset from the iris center.

If

$$\overrightarrow{X_p}^{\overrightarrow{WA}}$$

are the coordinates of the center of the pupil in an image. Let

$$\overrightarrow{X_i^{WA}}$$

be the center of the iris in the image. Let

$$\overrightarrow{X_p^{LASER}}$$

be the center of the pupil in the laser's camera image. Let

$$\overrightarrow{X_i^{LASER}}$$

be the center of the iris in the laser's camera image. Even if the iris or pupil are not circular (e.g. elliptical) there will still be a center for each of the pupil and iris. Then, the center position $\vec{c}$ with respect to pupil center for the surgery can be defined as:

$$\overrightarrow{C} = - \overrightarrow{X_i^{WA}} + \overrightarrow{X_p^{WA}} - \overrightarrow{X_p^{LASER}} + \overrightarrow{X_i^{LASER}}$$

As disclosed herein, features consistent with the present embodiments may be implemented via computer-hardware, software and/or firmware. For example, the systems and methods disclosed herein may be embodied in various forms including, for example, a data processor, such as a computer that also includes a database, digital electronic circuitry, firmware, software, computer networks, servers, or in combinations of them. Further, while some of the disclosed implementations describe specific hardware components, systems and methods consistent with the innovations herein may be implemented with any combination of hardware, software and/or firmware. Moreover, the above-noted features and other aspects and principles of the innovations herein may be implemented in various environments. Such environments and related applications may be specially constructed for performing the various routines, processes and/or operations according to the embodiments or they may include a general-purpose computer or computing platform selectively activated or reconfigured by code to provide the necessary functionality. The processes disclosed herein are not inherently related to any particular computer, network, architecture, environment, or other apparatus, and may be implemented by a suitable combination of hardware, software, and/or firmware. For example, various general-purpose machines may be used with programs written m accordance with teachings of the embodiments, or it may be more convenient to construct a specialized apparatus or system to perform the required methods and techniques.

It should also be noted that the various logic and/or functions disclosed herein may be enabled using any number of combinations of hardware, firmware, and/or as data and/or instructions embodied in various machine-readable or computer-readable media, in terms of their behavioral, register transfer, logic component, and/or other characteristics. Computer-readable media in which such formatted data and/or instructions may be embodied include, but are not limited to, non-volatile storage media in various forms (e.g., optical, magnetic or semiconductor storage media) and carrier waves that may be used to transfer such formatted data and/or instructions through wireless, optical, or wired signaling media or any combination thereof. Examples of transfers of such formatted data and/or instructions by carrier waves include, but are not limited to, transfers (uploads, downloads, e-mail, etc.) over the Internet and/or other computer networks via one or more data transfer protocols (e.g., HTTP, FTP, SMTP, and so on).

Unless the context clearly requires otherwise, throughout the description and the claims, the words "comprise," "comprising," and the like are to be construed in an inclusive sense as opposed to an exclusive or exhaustive sense; that is to say, in a sense of "including, but not limited to." Words using the singular or plural number also include the plural or singular number respectively. Additionally, the words "herein," "hereunder," "above," "below," and words of similar import refer to this application as a whole and not to any particular portions of this application. When the word "or" is used in reference to a list of two or more items, that word covers all of the following interpretations of the word: any of the items in the list, all of the items in the list and any combination of the items in the list.

Although certain presently preferred implementations of the invention have been specifically described herein, it will be apparent to those skilled in the art to which the invention pertains that variations and modifications of the various implementations shown and described herein may be made without departing from the scope of the invention. Accordingly, it is intended that the invention be limited only to the extent required by the applicable rules of law.

The foregoing description, for purpose of explanation, has been described with reference to specific examples.

However, the illustrative discussions above are not intended to be exhaustive or to limit the invention to the precise forms disclosed. Many modifications and variations are possible in view of the above teachings. The examples were chosen and described in order to best explain the principles of the invention and its practical applications, to thereby enable others skilled in the art to best utilize the invention and various examples with various modifications as are suited to the particular use contemplated. The invention is defined in the appended set of claims.

**Claims**

1. A system for aligning an eye for laser surgery, comprising:

   a pupilometer (40) with an imaging device configured to obtain a plurality of images of the eye from which the pupilometer can determine changes in pupil size;
   a variable illumination source (30);
   a computer with a processor (20) and memory in communication with the pupilometer, such that the processor can receive pupil size signals from the pupilometer;
   wherein the computer is configured to determine a desired optical light output sufficient to affect changes in pupil size as determined from the received pupil size signals; and
   send control signals to the variable illumination source (30) to obtain the desired optical light output to the eye, wherein the desired optical light output are both photopic and scotopic,
   wherein the computer is further configured to, determine a pupil center and an iris center for a scotopic image measure a pupil center and an iris center of a photopic image determine a pupil center and a cyclotorsional (CT) angle offset between the scotopic and photopic images.

2. The system of claim 1 wherein the computer is further configured to send photopic and scotopic images obtained by the imaging device to a data storage to be analyzed by the processor to find the pupil center and the CT angle offset.

3. The system of claim 1, wherein the pupilometer comprises an optical sensor (42) and a computer processor (44).

4. The system of claim 3, wherein the processor of the pupilometer and the processor of the computer system are the same processor.

5. The system of claim 5, wherein the computer comprises a dynamic feedback mechanism configured to direct optical light output in response to changes in pupil size as determined by the processor from the received pupil size signals.

**Patentansprüche**

1. System zum Ausrichten eines Auges zur Laserchirurgie, umfassend:

   ein Pupillometer (40) mit einer Bildgebungsvorrichtung, die ausgestaltet ist, um eine Vielzahl von Bildern des Auges zu erhalten, woraus das Pupillometer Änderungen der Pupillengröße bestimmen kann;
   eine variable Beleuchtungsquelle (30);
   einen Computer mit einem Prozessor (20) und Speicher in Kommunikation mit dem Pupillometer, so dass der Prozessor Pupillengrößensignale von dem Pupillometer empfangen kann;
   wobei der Computer ausgestaltet ist zum Bestimmen einer gewünschten optischen Lichtausgabe, die ausreichend ist, um Änderungen in der Pupillengröße gemäß Bestimmung aus den empfangenen Pupillengrößensignalen zu bewirken; und
   Senden von Steuersignalen an die variable Beleuchtungsquelle (30), um die gewünschte optische Lichtausgabe an das Auge zu erhalten,
   wobei die gewünschte optische Lichtausgabe sowohl photopisch als auch skotopisch ist, wobei der Computer des Weiteren ausgestaltet ist zum Bestimmen eines Pupillenzentrums und eines Iriszentrums für ein skotopisches Bild,
   Messen eines Pupillenzentrums und eines Iriszentrums eines photopischen Bildes,
   Bestimmen eines Pupillenzentrums und eines Zyklotorsions- (CT)-Winkelversatzes zwischen dem skotopischen und dem photopischen Bild.

2. System nach Anspruch 1, wobei der Computer des Weiteren ausgestaltet ist, um photopische und skotopische Bilder, die durch die Bildgebungsvorrichtung erhalten wurden, an einen Datenspeicher zu senden, um durch den Prozessor analysiert zu werden, um das Pupillenzentrum und den CT-Winkelversatz zu finden.

3. System nach Anspruch 1, wobei das Pupillometer einen optischen Sensor (42) und einen Computerprozessor (44) umfasst.

4. System nach Anspruch 3, wobei der Prozessor des Pupillometers und der Prozessor des Computersystems derselbe Prozessor sind.

5. System nach Anspruch 5, wobei der Computer einen dynamischen Rückmeldemechanismus umfasst, der ausgestaltet ist, um optische Lichtausgabe in Reaktion auf Änderungen der Pupillengröße, wie sie durch den Prozessor aus den empfangenen Pupillengrößensignalen bestimmt wurde, zu lenken.

**Revendications**

1. Système pour l'alignement d'un œil pour une chirurgie au laser, comprenant :

un pupillomètre (40) doté d'un dispositif d'imagerie conçu pour obtenir une pluralité d'images de l'œil à partir desquelles le pupillomètre peut déterminer des changements de taille de la pupille ;
une source d'éclairage variable (30) ;
un ordinateur doté d'un processeur (20) et d'une mémoire en communication avec le pupillomètre, de telle sorte que le processeur puisse recevoir des signaux de taille de pupille en provenance du pupillomètre ;
dans lequel l'ordinateur est configuré pour déterminer une sortie de lumière optique souhaitée suffisante pour affecter des changements de taille de la pupille tels que déterminés à partir des signaux de taille de pupille reçus ; et
envoyer des signaux de commande à la source d'éclairage variable (30) pour obtenir la sortie de lumière optique souhaitée vers l'œil,
dans lequel la sortie de lumière optique souhaitée est à la fois photopique et scotopique,
dans lequel l'ordinateur est en outre configuré pour,

déterminer un centre de pupille et un centre d'iris pour une image scotopique
mesurer un centre de pupille et un centre d'iris d'une image photopique
déterminer un centre de pupille et un décalage d'angle de cyclotorsion (CT) entre les images scotopique et photopique.

2. Système selon la revendication 1 dans lequel l'ordinateur est en outre configuré pour envoyer des images photopiques et scotopiques obtenues par le dispositif d'imagerie à un stockage de données pour être analysées par le processeur afin de trouver le centre de la pupille et le décalage d'angle CT.

3. Système selon la revendication 1, dans lequel le pupillomètre comprend un capteur optique (42) et un processeur informatique (44).

4. Système selon la revendication 3, dans lequel le processeur du pupillomètre et le processeur du système informatique sont le même processeur.

5. Système selon la revendication 5, dans lequel l'ordinateur comprend un mécanisme de rétroaction dynamique conçu pour diriger une sortie de lumière optique en réponse à des changements de taille de la pupille tels que déterminés par le processeur à partir des signaux de taille de pupille reçus.

FIG. 1

FIG. 1A

FIG. 1B

FIG. 1C

FIG. 1D

FIG. 1E

FIG. 2

FIG. 3A

FIG. 3B

FIG. 4A

FIG. 4B

FIG. 5

# Difference of pupil size

FIG. 6

EP 3 509 546 B1

| Obtaining scotopic image of the eye | 710 |

↓

| Determining a pupil center and iris center | 712 |

↓

| Obtaining photopic image of the eye | 714 |

↓

| Determining a pupil center and iris center | 716 |

↓

| Determining pupil center and CT angle offset of the photopic to the scotopic image | 718 |

↓

| Transferring the photopic image, pupil center and CT angle offset to the laser system | 719 |

↓

| Capturing a treatment image of the eye using a camera in the laser system | 720 |

↓

| Determining a pupil center and iris center | 722 |

↓

| Determining pupil center and CT angle offset of the treatment image to the photopic image | 724 |

↓

| Converting the pupil center and CT angle offset of the treatment image to the scotopic image | 723 |

↓

| Aligning the laser system with the eye using the determined pupil center and CT angle offset of the treatment image to the scotopic image | 726 |

FIG. 7

| Obtaining scotopic image of the eye | 810 |

↓

| Obtaining a photopic image of the eye | 812 |

↓

| Transferring the scotopic and photopic images of the eye to the laser system | 814 |

↓

| Obtaining a treatment image of the eye | 816 |

↓

| Determining pupil center and iris center of the scotopic image | 818 |

↓

| Determining pupil center and iris center of the photopic image | 820 |

↓

| Determining pupil center and iris center of the treatment image | 822 |

↓

| Determining pupil center and CT angle offset of the photopic image to the scotopic image | 824 |

↓

| Determining pupil center and CT angle offset of the treatment image to the photopic image | 826 |

↓

| Converting the pupil center and CT angle offset of the treatment image to the scotopic image | 828 |

↓

| Aligning the laser system with the eye using the determined pupil center and CT angle offset of the treatment image to the scotopic image | 830 |

FIG. 8

Obtaining a first image of an eye     **910**

Locating the pupil and radius in the first image of the eye **912**

Extracting a fixed or variable width iris ring     **914**

Unwrapping the iris ring and dividing into a
fixed number of sectors     **916**

Locating a salient region in each sector in the
first image of the eye     **918**

Extracting the properties of each of the salient regions **920**

Obtaining a second image of the eye     **922**

Locating the salient region of the second image of the eye **924**

Estimating the angular displacement for each of the salient regions and
estimating the total torsional angle estimation     **926**

FIG. 9

**EP 3 509 546 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 7044602 B
- US 20120242956 A1
- US 7708405 B
- US 6322216 B
- US 2012242956 A1
- US 2015148788 A1
- US 6315413 B
- US 5713892 A
- US 5646791 A
- US 4665913 A
- US 4669466 A
- US 4732148 A
- US 4770172 A
- US 4773414 A
- US 5207668 A
- US 5108388 A
- US 5219343 A
- US 5163934 A
- US 6004313 A
- US 6095651 A
- US 6271915 B